# EUROPÄISCHE PATENTANMELDUNG

(11) **EP 0 684 021 A1**
(43) Veröffentlichungstag der Anmeldung: **29.11.1995**
(21) Anmeldenummer: 94108073.1
(22) Anmeldetag: 26.05.1994
(51) Int. Cl.: A61F 2/06

(54) **Stent mit ineinander eingreifenden Filamenten**

(71) Anmelder: TFX Medical, Annacotty, County Limerick (IR)
(72) Erfinder: Amstrup, Mogens Dipl.-Ing, D-73614 Schorndorf (DE)
(74) Vertreter: KOHLER SCHMID + PARTNER

(57) **Zusammenfassung**

Ein selbstexpandierender Stent für Hohlorgane wird zur Schienung und/oder zum Offenhalten eines Hohlorgans vorgestellt. Der Stent besteht aus Bändern 11, die ein tubuläres und selbstexpandierendes Netz aufspannen. In einem kollabierten Zustand des Lumens wird das Netz in Achsrichtung gestreckt und in einem ausgedehnten Zustand des Lumens in Achsrichtung verkürzt und das Netz ist aus mindestens zwei Bändern 11 aufgebaut. Erfindungsgemäß wird jedes einzelne Band 11 aus mindestens zwei parallel zueinander verlaufenden Filamenten 13 hergestellt, die punktuell miteinander verbunden sind und die an Kreuzbereichen 12 ineinandergreifen.

## Beschreibung

Gegenstand der Erfindung ist ein Stent zur Schienung und/oder zum Offenhalten eines Hohlorgans, mit aus Filamenten bestehendem tubulärem und selbstexpandierbarem Netz, das im kollabierten Zustand des Lumens in Achsrichtung gestreckt und im ausgedehnten Zustand des Lumens in Achsrichtung verkürzt ist, und das aus mindestens zwei Bändern aufgebaut ist.

Ein derartiger Stent ist in der europäischen Anmeldung EP 94 106 490.9 beschrieben.

Bei Patienten mit erhöhtem chirurgischem Risiko ist die transluminale Applikation von Stents zur Schienung oder permanenten Dehnung von Stenosen oder andere Formen von Lumeneinschränkungen von Hohlorganen besonders vorteilhaft. Chirurgische und diagnostische Eingriffe können mit Hilfe der interventionellen Radiologie und mit Hilfe verschiedener visueller diagnostischer Möglichkeiten ergänzt oder im günstigsten Fall überflüssig werden. Durch die transluminale Implantation von Stents ist es möglich, ein Gefäßlumen oder ein Hohlorgan permanent offen zu halten.

Der in der obengenannten EP-Patentanmeldung beschriebene Stent ist aufgebaut aus Bändern, die im Querschnitt rechteckförmig ausgebildet und an Kreuzungspunkten des Stents miteinander fixiert sind. Der bekannte Stent weist im kollabierten Zustand einen kleineren Durchmesser auf als ein selbstexpandierender Stent aus Rundfilamenten und der im Querschnitt rechteckförmig ausgebildete Stent kann aus einer geringeren Anzahl von Bändern hergestellt werden. Die Verbindung der Bänder an den Kreuzungspunkten wird dadurch erreicht, daß die Bänder an den Kreuzungspunkten vollkommen ummantelt sind. Innerhalb der ummantelten Schicht können sich die Bänder zueinander gelenkig bewegen. Je nachdem, welches Material für die Ummantelung ausgewählt wird, kann eine Rückstellkraft aus dem kollabierten Zustand des Stents zum ausgedehnten Zustand verstärkt werden. Die Bänder liegen an den Kreuzungspunkten über ihren metallischen Flächen aneinander an und die sich kreuzenden Bänder sind gesamthaft kunststoffummantelt, so daß ein fixiertes Gelenk im Kreuzungspunkt gebildet wird.

Der Erfindung liegt die Aufgabe zugrunde, einen selbstexpandierenden Stent der eingangs genannten Art dahingehend weiterzubilden, daß eine möglichst gleichmäßige Anlage der Filamente an der Innenwand eines Hohlorgans gewährleistet ist, daß die Rückstellkräfte des Stents über die Kreuzungspunkte ausdehnbar sind und daß eine sichere und stabile Anwendung des Stents durch eine Verbesserung der Gelenkwirkung der Kreuzpunkte erreicht wird.

Diese Aufgabe wird erfindungsgemäß dadurch gelöst, daß jedes einzelne Band aus mindestens zwei parallel zueinander verlaufenden, punktuell miteinander verbundenen Filamenten besteht, die an Kreuzungsbereichen ineinandergreifen.

Das Aufspalten des Bands in mindestens zwei parallel verlaufende punktuell miteinander verbundene Filamente, die an den Kreuzungspunkten geflechtmäßig ineinandergreifen, erlaubt die Herstellung des Stents mit Kreuzungsbereichen, die nicht mehr mit einer Beschichtung versehen werden müssen, um ein Gelenk an dem Kreuzungspunkt herzustellen. Demzufolge ist der Kreuzungspunkt in einen Kreuzungsbereich umgewandelt, wobei die punktuellen Kräfte des Kreuzungspunkts gewebeschonend über dem Kreuzungsbereich verteilt sind. Zudem ist ein Nachlassen der Rückstellkraft durch eine Ermüdung des Beschichtungsmaterials an den Kreuzpunkten nicht mehr möglich, weil das an den Kreuzungspunkten ausgebildete Gelenk nicht mehr anhand einer Beschichtung zusammengehalten wird, sondern durch das aus mindestens zwei Filamenten bestehende ineinandergreifende Geflecht der Bänder gebildet ist.

In bevorzugter Ausgestaltung der Erfindung ist die Querschnittsform der Filamente rechteckförmig. Diese Ausführungsform hat den Vorteil, daß der Stent einen kleineren Durchmesser aufweisen kann, wie z.B. ein selbstexpandierender Stent aus Rundfilamenten. Damit liegt der erfindungsgemäße Stent flächenhaft und damit gebeweschonend an der Innenwand eines Hohlorgans und die Filamente drücken nicht linienförmig auf das angrenzende Gewebe. Die Lage des einzelnen Filaments kann über die gesamte Länge des Stents optisch einfachst festgestellt werden. Wird für die Herstellung des erfindungsgemäßen Stents ein Stahlflachband benutzt, das wesentlich breiter ist als hoch, so ist der Stent im gestreckten Zustand sehr dünn und kann über sehr dünne Einführungsinstrumente sicher durch das Lumen des Hohlorgans eingeführt und plaziert werden. Weiterhin weist der erfindungsgemäße Stent in dieser Ausführungsform eine große Variabilität im Durchmesser, in der Länge und in der Expansionsstärke bei großer longitudinaler Flexibilität auf. Dies wird dadurch erreicht, daß bei einem Flachband die unterschiedlich gerichteten Kraftkomponenten auch unterschiedlich groß sind, im Gegensatz zu einem runden Draht, bei dem die aus ihm resultierenden Kraftkomponenten immer gleich groß sind. Der erfindungsgemäße Stent kann sich in dieser Ausführungsform den unterschiedlichsten Verläufen der Hohlorgane besser anpassen. Durch eine flächenhafte Anlage an der Innenwandung des zu dehnenden Hohlorgans kann auch die Druckbelastung und eine eventuelle überschießende intimale Hyperplasie bzw. Granulationsbildung im Bereich der Stentenden reduziert werden.

In einer weiteren Ausgestaltung der Erfindung enden die Filamente an den freien Enden des Stents parallel verlaufend und sind miteinander verbunden. Dies hat den Vorteil, daß die freien Enden des Stents keine scharfen Ecken oder Kanten aufweisen können, die das Gewebe verletzten könnten oder die auch zu einer problematischen Entfernung des Stents beitragen könnten. Durch diese Fixierung der Enden ist gewährleistet, daß das Lumen nur einen geringgradig vergrößerten Durchmesser an den freien Enden aufweist, jedoch für eine ausreichende Fixation des Stents sorgt.

In einer Weiterbildung dieser Ausführungsform weisen die freien Enden des Stents Abrundungen auf. Diese Maßnahme hat den Vorteil, daß ein schonendes Anliegen des Stents auch im Endbereich am Gewebe stattfindet, wobei eine mögliche Verletzung oder Irritation des belasteten Gewebes durch den Endbereich des Stents vermieden werden kann.

Der erfindungsgemäße Stent kann auch Filamente aufweisen, die zumindest teilweise mit einer Ummantelung versehen sind. Diese Maßnahme hat den Vorteil, daß eine mögliche erwünschte Beschichtung oder Oberfläche auch auf die Filamente gebracht werden kann, wodurch eine verbesserte Biokompabilität des Stents erreicht werden kann oder eine besondere gewebeschonende Wirkung erzielbar ist. Neben einer gummielastischen Beschichtung oder einer Beschichtung aus Kunststoff können auch hydrophile oder antithrombogene Beschichtungen auf den Bändern vorgesehen werden. Eine hydrophile Beschichtung ermöglicht verbesserte Durchflußwerte an den ausgedehnten Stellen im Hohlorgan.

In einer anderen Ausführungsform sind die Filamente jedes Bandes vor und hinter den Kreuzungsbereichen punktuell miteinander verbunden. Diese Maßnahme hat den Vorteil, daß die Kreuzungsbereiche mechanisch stabil gehalten und definiert sind, damit im kollabierten sowie im ausgedehnten Zustand die Rückstellkräfte an den Kreuzungspunkten vorteilhafterweise verteilt werden und reproduzierbar sind. Durch diese Anordnung wird eine stabile mechanische Anordnung erreicht.

Die punktuellen Verbindungen können durch Schweißpunkte oder dergleichen hergestellt werden. Dies hat den Vorteil, daß bewährte Schweißtechniken zur Herstellung des erfindungsgemäßen Stents eingesetzt werden können, die eine effektive und kostengünstige sowie eine sichere und zuverlässige Verbindung der zu einem Band zusammengefügten Filamente ermöglichen.

Bei einer weiteren Ausführungsform der Erfindung sind die Filamente aus Memory-Werkstoff hergestellt. Die Benutzung von beispielsweise einer Memory-Legierung, z.B. Nitinoldraht oder -band erlaubt beispielsweise, daß der Stent über ein bekanntes Einführungsinstrument an die entsprechenden Gefäßstellen geführt und in situ expandiert werden kann und dabei eine werkstoffgespeicherte Endform annehmen kann.

Es ist auch möglich, den Stent aus Kunststoff oder Metall zu produzieren. Dadurch kann je nach Anforderung die Biokompatibilität des Stents und/oder die gewebeschonende Wirkung verbessert werden.

In einer weiteren Ausführungsform der Erfindung weist das Netz auf der Außenfläche eine Oberfläche auf, die erhöhte Reibungswerte erzeugt. Diese Maßnahme hat den Vorteil, daß der Stent nach Einführung in das Hohlorgan im Hohlorgan einen sicheren, unverrückbaren Sitz findet.

Weiterhin kann bei Bedarf der gesamte Stent kunststoff- oder wirkstoffummantelt sein. Dabei ergeben sich Ausführungsformen, bei denen die Ummantelung die Freiräume zwischen den Bändern abdeckt.

Weitere Vorteile ergeben sich aus der Beschreibung und der beigefügten Zeichnung. Ebenso können die vorstehend genannten und die noch weiter ausgeführten Merkmale erfindungsgemäß jeweils einzeln oder in beliebiger Kombination miteinander verwendet werden. Die erwähnten Ausführungsformen sind nicht als abschließende Aufzählung zu verstehen, sondern haben vielmehr beispielhaften Charakter. Die Erfindung ist in der Zeichnung dargestellt und wird anhand von Ausführungsbeispielen erläutert. Es zeigt:
- Fig. 1: einen erfindungsgemäßen Stentabschnitt in ausgedehntem Zustand mit Freiräumen und einem Netz, das aus acht Bändern besteht;
- Fig. 2: einen Abschnitt eines erfindungsgemäßen Stents mit sechs Bändern;
- Fig. 3: eine vergrößerte Darstellung eines Kreuzungsbereiches im ausgedehnten Zustand;
- Fig. 4: eine expandierte Darstellung des Kreuzungsbereiches im kollabierten Zustand;
- Fig. 5: einen Endabschnitt eines Stents im Bereich eines freien Endes;
- Fig. 6: einen Abschnitt eines aus zwei Filamenten bestehenden einzelnen Bandes in gerader Erstreckung;
- Fig. 7: einen Querschnitt gemäß VII-VII der Fig. 6;
- Fig. 8: einen Abschnitt eines einzelnen Bandes mit zwei runden Filamenten;
- Fig. 9: einen Endabschnitt eines Stents im Bereich eines freien Endes.

Die einzelnen Figuren der Zeichnung zeigen den erfindungsgemäßen Gegenstand teilweise stark schematisiert und sind nicht maßstäblich zu verstehen. Die Gegenstände der einzelnen Figuren sind stark vergrößert dargestellt, damit ihr Aufbau besser gezeigt werden kann.

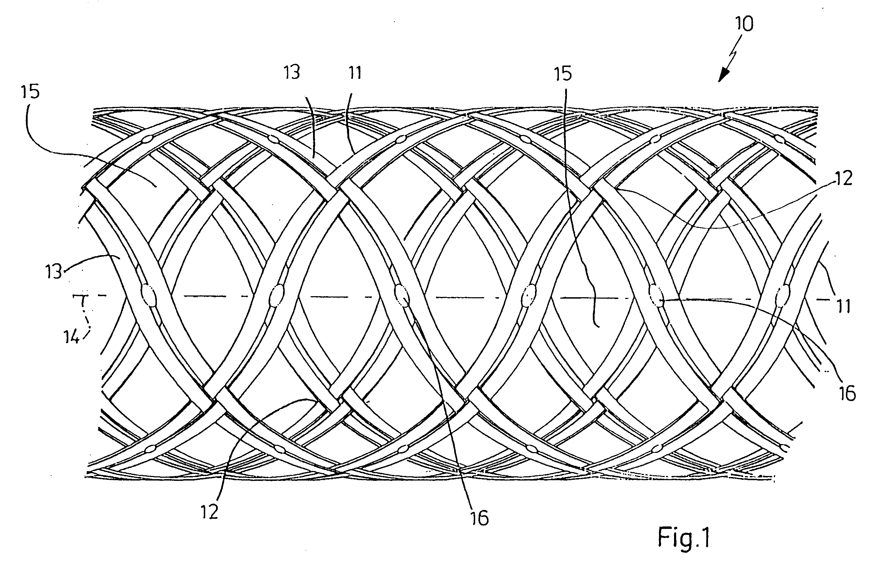

Fig. 1 zeigt ein Stentabschnitt 10 eines aus acht Bändern 11 bestehenden Stents in weitgehend ausgedehntem Zustand des Lumens. Die helixförmigen Bänder 11 begrenzen ein zylindrisches Volumen und gestalten ein geflochtenes Netz, das einen im wesentlichen zylindrischen Hohlkörper definiert. Jedes Band 11 besteht aus zwei benachbarten zueinander parallel verlaufenden Filamenten 13 und die Bänder 11 kreuzen sich an Kreuzungsbereichen 12, wobei die Filamente 13 geflechtmäßig ineinandergreifen. Das Netz läuft um eine zentrale Symmetrieachse 14 in einer longitudinalen Richtung. Das aus den Bändern 11 gestaltete Netz weist Freiräume 15 auf, deren Größe davon abhängt, wieviel Bänder 11 benutzt wurden, um das Netz zu erstellen, sowie ob sich das Netz im kollabierten Zustand des Lumens oder im ausgedehnten Zustand des Lumens befindet. Die jeweils zu einem Band 11 gehörenden Filamente 13 sind mittels Verbindungen 16 zusammengehalten. Die Verbindungen 16 sind hinter und vor den Kreuzungsbereichen 12 derart angeordnet, daß ein stabiler Kreuzungsbereich 12 gewährleistet ist, der große Rückstellkräfte beweglich aufnehmen kann.

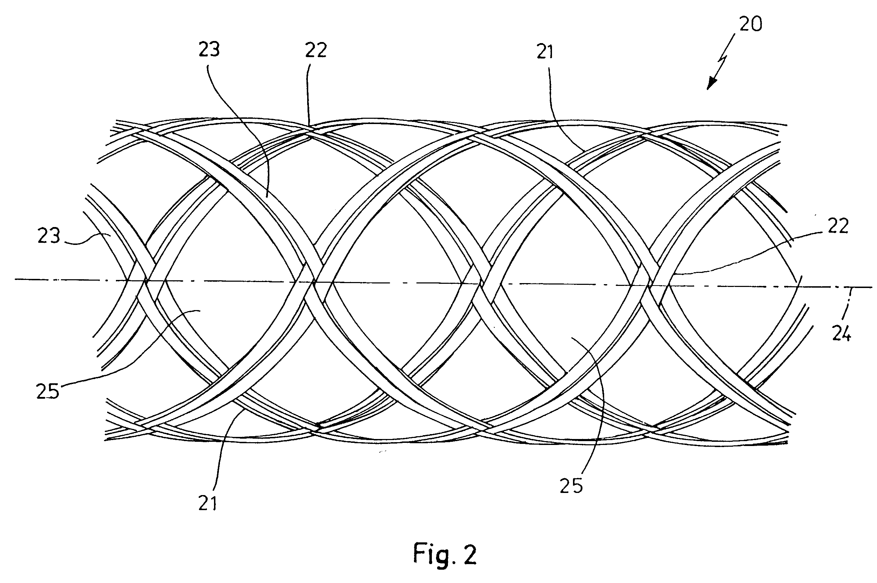

Fig. 2 zeigt einen erfindungsgemäßen Stentabschnitt 20 eines aus sechs Bändern 21 bestehenden Stents. Jedes Band 21 besteht aus zwei parallel zueinander verlaufenden Filamenten 23, die in Kreuzungsbereichen 22 in die Filamente 23 von einem anderen Band 21 eingreifen. Die gemäß Fig. 2 von sechs Bändern 21 gestalteten Filamente 23 definieren ein zylindrisches, um eine Zentralachse 24 verlaufendes Volumen, das vom geflochtenen Netz umhüllt ist. Das Netz gemäß Fig. 2 ist im ausgedehnten Zustand des Lumens gezeigt. Bei dem aus sechs Bändern 21 bestehenden Stent gemäß Fig. 2 sind Freiräume 25 erstellt worden, die gegenüber denen des Ausführungsbeispiels gemäß Fig. 1 eine andere Größe und Form aufweisen können.

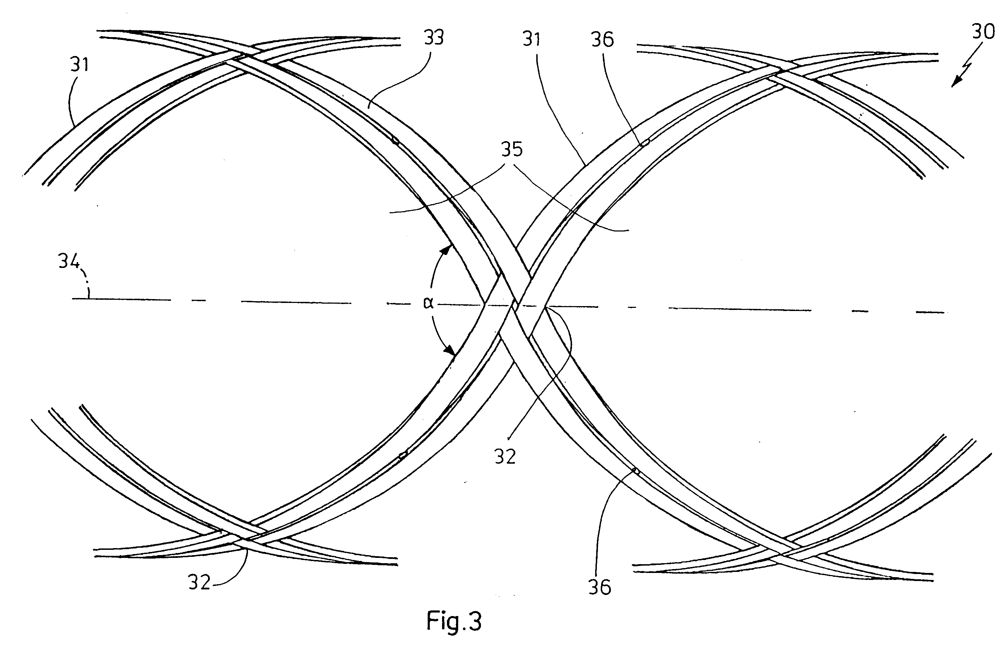

Fig. 3 zeigt einen vergrößerten Stentabschnitt 30 eines aus vier Bändern 31 bestehenden Stents im ausgedehnten Zustand des Lumens. Die Bänder 31 sind jeweils aus zwei Filamenten 33 hergestellt, die sich überlappend an Kreuzungsbereichen 32 ineinander eingreifen. Das mit vier Bändern versehene Ausführungsbeispiel gemäß Fig. 3 gestaltet ein zylinderförmiges Lumen, das symmetrisch um eine zentrale Achse 34 verläuft. Das Netz weist Freiräume 35 auf, deren Größe und Form sich auch im ausgedehnten Zustand des Lumens von der Größe und Form der Freiräume, die aus mit acht Bändern 11 aufgebauten Stents gemäß Fig. 1 bzw. dem sechs Bänder 21 aufweisenden Stent gemäß Fig. 2 gebildet werden, unterscheiden können. Die jeweiligen Filamente 36 jedes Bandes 31 sind mittels Verbindungen 36 zusammengehalten, wobei die Verbindungen 36 vor und hinter den Kreuzungsbereichen 32 so angeordnet sind, daß ein stabiler Kreuzungsbereich 32 geschaffen wird. Im ausgedehnten Zustand des Lumens weist der Kreuzungsbereich 32 zwischen den zwei ineinandergreifenden Bändern 31 einen Kreuzungswinkel α auf.

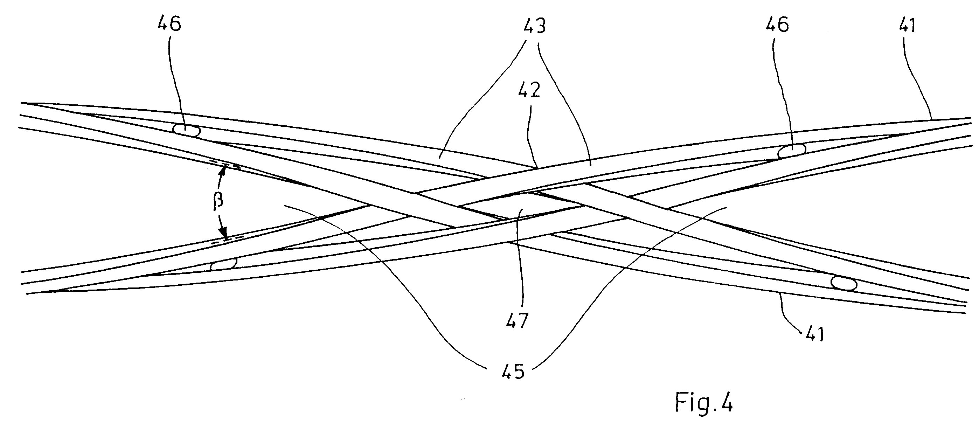

In Fig. 4 wird ein Ausschnitt eines Kreuzungsbereichs 42 eines erfindungsgemäßen Stents im kollabierten Zustand des Lumens gezeigt. In diesem Zustand ist der Stent axial verstreckt, wobei das Lumen kollabiert ist. Die jeweils aus zwei Filamenten 43 bestehenden zwei Bänder 41 kreuzen sich überlappend im Kreuzungsbereich 42. Vor und hinter dem Kreuzungsbereich sind Verbindungen 46, die auch Schweißnaht-Eigenschaften aufweisen können, hergestellt. Im kollabierten Zustand weisen die sich im Kreuzungsbereich 42 kreuzenden Bänder 41 einen Kreuzungswinkel β auf, der deutlich kleiner ist als der Kreuzungswinkel im ausgedehnten Zustand des Lumens (Winkel α der Fig. 3) und die Freiräume 45 des Netzes sind entsprechend reduziert. Im kollabierten Zustand des Lumens wird ein Kreuzungsspalt 47 durch die ineinandergreifenden Filamente 43 der zwei Bänder 41 geformt, der eine quaderförmige Fläche bildet. Im Gegensatz zu den Freiräumen 45, die im allgemeinen eine kleinere Fläche im kollabierten Zustand als im ausgedehnten Zustand des Lumens aufweisen, können die Kreuzungsspalte 47 auch größere Freiflächen im kollabierten als im ausgedehnten Zustand aufweisen. In dieser Weise ist es möglich, den Kreuzungsbereich 42 im kollabierten Zustand über eine noch größere Fläche zu verteilen als im ausgedehnten Zustand des Lumens, wodurch eine größere Speicherung von Rückstellungsenergie in den Kreuzungsbereich 42 erreicht und ein stabiler Kreuzungsbereich 42 geschaffen wird. Die Ausbildung eines Kreuzungsspalts 47 im kollabierten Zustand des Lumens bewirkt eine Ausspaltung der jeweils zu einem Band 41 gehörigen Filamente 43, die im Gegensatz zum ausgedehnten Zustand, wo die parallel laufenden Filamente 43 eines Bandes 41 sich weitgehend in einer gemeinsamen Ebene befinden, sich leicht auseinanderspreizen, wobei anhand der Verbindungen 46 Rückstellungskräfte in den Filamenten 43 federgelagert werden.

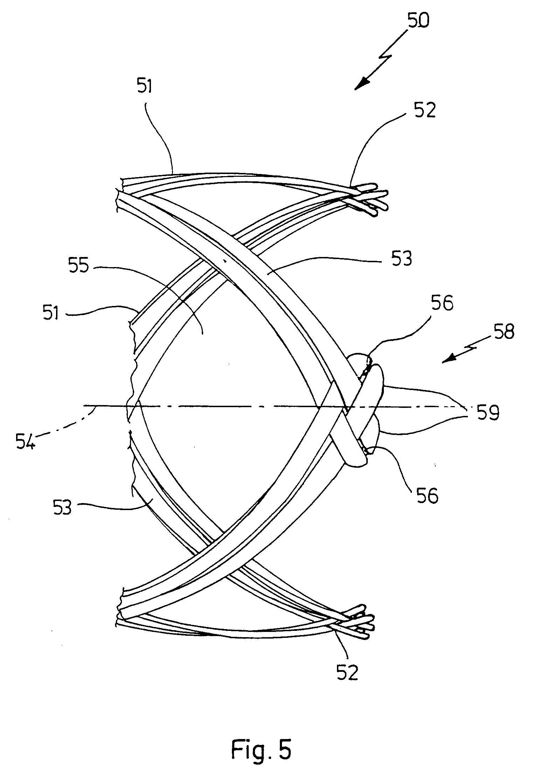

Fig. 5 zeigt einen Stentabschnitt 50 im Bereich des freien Endes 58. Das symmetrisch um eine Achse 54 verlaufende Netz ist aus sechs Bändern 51 aufgebaut und jedes Band 51 besteht aus zwei Filamenten 53. Das freie Ende 58 des Stents beginnt unmittelbar nach den Kreuzungsbereichen 52. Die sich kreuzenden Bänder 51 definieren Freiräume 55. Verbindungen 56 sind direkt nach den Kreuzungsbereichen 52 vorgesehen, die die zwei Filamente 53 eines Bandes 51 zueinander parallel verlaufend auch am freien Ende 58 des Stents abschließend zusammenhalten. Am freien Ende 58 weisen die Filamente 53 Abrundungen 59 auf, die ein schonendes Anliegen am Gewebe des Hohlorgans gewährleisten.

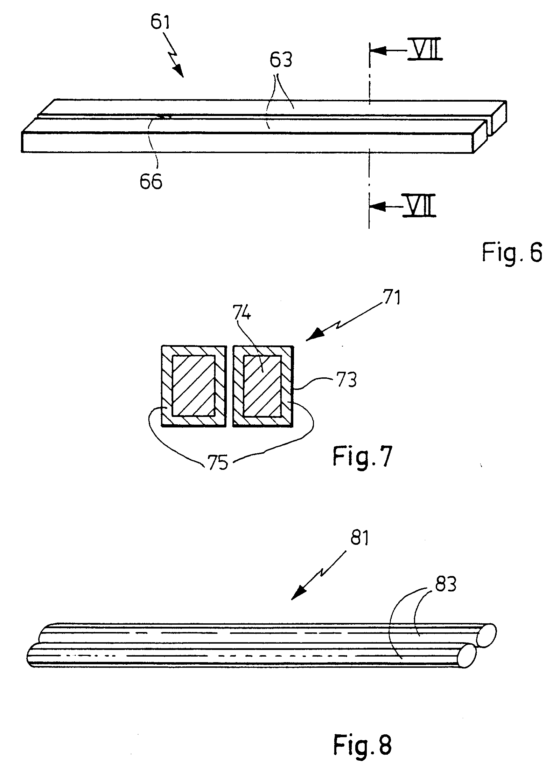

Fig. 6 zeigt einen Abschnitt eines Bandes 61 aus zwei parallel verlaufenden Filamenten 63 jeweils mit rechteckigem Querschnitt. Die Filamente 63 sind mittels einer Schweißstelle bzw. -verbindung aneinandergehalten.

Fig. 7 zeigt einen Querschnitt durch das Segment der Fig. 6 gemäß dem Schnitt VII-VII. Das im Querschnitt gezeigte Band 71 besteht aus zwei jeweils rechteckigen Filamenten 73 und jedes Filament 73 weist einen Kern 74 und eine Ummantelung 75 auf, wobei die Ummantelung aus gewebeschonendem Material hergestellt sein kann.

Schließlich zeigt die Fig. 8 eine Ausführungsform des Bandes 81, worin das Band 81 aus drahtförmigen Filamenten 83 mit rundem Querschnitt besteht.

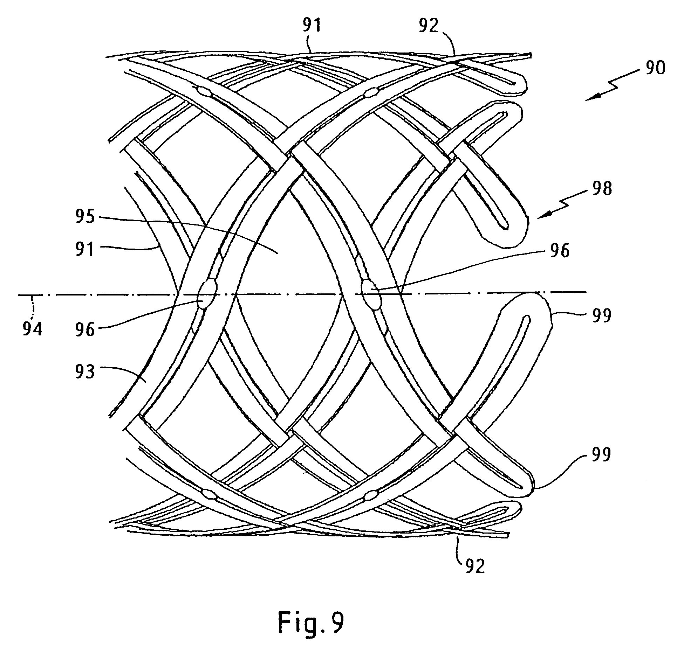

Fig. 9 zeigt ein anderes Ausführungsbeispiel eines Stentabschnitts 90 im Bereich des freien Endes 98. Das symmetrisch um eine Achse 94 verlaufende Netz besteht aus acht Bändern 91 und jedes Band 91 ist aus zwei Filamenten 93 aufgebaut. Das freie Ende 98 des Stents beginnt nach den Kreuzungsbereichen 92 und die sich kreuzenden Bänder 91 definieren Freiräume 95. Verbindungen 96 sind direkt vor den Kreuzungsbereichen 92 vorgesehen, die die zwei Filamente 93 eines Bandes 91 zueinander parallel verlaufend auch am freien Ende 98 des Stents zusammenhalten. Am freien Ende 98 weisen die Filamente 93 abschließende Abrundungen 99 auf, die ein schonendes Anliegen am Gewebe des Hohlorgans gewährleisten.

Ein selbstexpandierender Stent für Hohlorgane wird zur Schienung und/oder zum Offenhalten eines Hohlorgans vorgestellt. Der Stent besteht aus Bändern 11, die ein tubuläres und selbstexpandierendes Netz aufspannen. In einem kollabierten Zustand des Lumens wird das Netz in Achsrichtung gestreckt und in einem ausgedehnten Zustand des Lumens in Achsrichtung verkürzt und das Netz ist aus mindestens zwei Bändern 11 aufgebaut. Erfindungsgemäß wird jedes einzelne Band 11 aus mindestens zwei parallel zueinander verlaufenden Filamenten 13 hergestellt, die punktuell miteinander verbunden sind und die an Kreuzbereichen 12 ineinandergreifen.

## Patentansprüche

1. Stent zur Schienung und/oder zum Offenhalten eines Hohlorgans, mit aus Bändern (11; 21; 31; 41; 51; 61; 71; 81) bestehendem tubulärem und selbstexpandierbarem Netz, das im kollabierten Zustand des Lumens in Achsrichtung (14; 24; 34; 54) gestreckt und im ausgedehnten Zustand des Lumens in Achsrichtung (14; 24; 34; 54) verkürzt ist und das aus mindestens zwei Bändern (11; 21; 31; 41; 51; 61; 71; 81) aufgebaut ist, dadurch gekennzeichnet, daß jedes einzelne Band (11; 21; 31; 41; 51; 61; 71; 81) aus mindestens zwei parallel zueinander verlaufenden punktuell miteinander verbundenen Filamenten (13; 23; 33; 43; 53; 63; 73; 83) besteht, die an Kreuzungsbereichen (12; 22; 32; 42; 52) ineinandergreifen.

2. Stent nach Anspruch 1, dadurch gekennzeichnet, daß die Querschnittsform der Filamente (13; 23; 33; 43; 53; 63; 73; 83) rechteckförmig ist.

3. Stent nach Anspruch 1 oder 2, dadurch gekennzeichnet, daß die Filamente (53) an freien Enden (58) des Stents (50) parallel verlaufend enden und miteinander verbunden sind.

4. Stent nach einem der vorangehenden Ansprüche, dadurch gekennzeichnet, daß die Filamente (73) zumindest teilweise einen mit einer Ummantelung (75) versehenen Kern (74) aufweisen.

5. Stent nach einem der vorangehenden Ansprüche, dadurch gekennzeichnet, daß die Filamente (13; 23; 33; 43; 53; 63; 73; 83) jedes Bandes (11; 21; 31; 41; 51; 61; 71; 81) vor und hinter den Kreuzungsbereichen (12; 22; 32; 42; 52) punktuell miteinander verbunden sind.

6. Stent nach einem der vorangehenden Ansprüche, dadurch gekennzeichnet, daß die Filamente (13; 23; 33; 43; 53; 63; 73; 83) über Schweißpunkte (16; 26; 36; 46; 56; 66) oder dergleichen miteinander verbunden sind.

7. Stent nach einem der vorangehenden Ansprüche, dadurch gekennzeichnet, daß die Filamente (13; 23; 33; 43; 53; 63; 73; 83) aus einem Memory-Werkstoff hergestellt sind.

8. Stent nach einem der vorangehenden Ansprüche, dadurch gekennzeichnet, daß die Filamente (13; 23; 33; 43; 53; 63; 73; 83) aus Metall oder Kunststoff bestehen.

9. Stent nach einem der vorangehenden Ansprüche, dadurch gekennzeichnet, daß das Netz auf der Außenfläche eine Oberfläche aufweist, die erhöhte Reibungswerte erzeugt.
